# EUROPEAN PATENT SPECIFICATION

(11) **EP 3 431 053 B1**
(45) Date of publication and mention of the grant of the patent: **08.02.2023**
(21) Application number: 16894500.4
(22) Date of filing: 12.09.2016
(51) Int. Cl.: A61F 2/966, A61F 2/07, A61M 25/00

(54) **THERAPEUTIC DEVICE**
THERAPEUTISCHE VORRICHTUNG
DISPOSITIF THÉRAPEUTIQUE

(30) Priority: 16.03.2016 JP 2016052624
(43) Date of publication of application: 23.01.2019
(73) Proprietor: Japan Lifeline Co., Ltd., Tokyo 140-0002 (JP)
(72) Inventor: HOSHIDA, Aki, Tokyo 140-0002 (JP); MORI, Kenji, Tokyo 140-0002 (JP)
(74) Representative: D Young & Co LLP
(86) International application number: PCT/JP2016/076835
(87) International publication number: WO 2017/158883

(56) References cited:
- WO-A1-2015/031025
- JP-A- S59 218 163
- JP-A- 2000 024 113
- JP-A- 2007 503 923
- US-A1- 2005 250 983
- US-A1- 2015 290 423

## Description

### Technical Field

The invention relates to a treatment apparatus that is to be used upon treatment, for example, of arterial dissection (a dissecting aneurysm), etc.

### Background Art

In recent years, in a case where a blood vessel or any other pipe-shaped organ in vivo is narrowed or occluded, a case where a dissecting aneurysm, etc. is present in a blood vessel, or the like, a technique (stent placement) has been used that makes it possible to expand or retain a lumen of a pipe-shaped organ or to prevent rupture of the aneurysm, etc. by placing a stent in an affected area. The stent includes a wire member (a wire), etc. In particular, a stent graft has been used upon treatment, for example, of arterial dissection, etc. in a blood vessel. The stent graft is provided with a tubular graft that covers the stent described above.

Now, an OSG (open stent graft) method has been proposed recently as one of treatment methods for arterial dissection in a thoracic aorta, etc. In the OSG method, the aorta is incised after a thoracic part is opened, and the stent graft is inserted from the incised part. Further, base end side of the stent graft is sutured to a vessel of a patient to make an anastomosis therebetween. Further, an anastomosis is made, on an as-needed basis, between the foregoing anastomosis part and another artificial blood vessel other than the foregoing stent graft.

Moreover, for example, a delivery catheter such as that disclosed in Patent Literature 1 is used in a procedure utilizing the above-described OSG method. The catheter is configured to be mounted with (to hold) a stent graft having a reduced diameter upon insertion of the stent graft from the foregoing incised part.

### Citation list

### Patent Literature

Patent Literature 1: Japanese Unexamined Patent Application Publication No. 2012-65998

US 2015/290423 A1 discloses a treatment apparatus comprising a catheter and a stent.

### Summary of Invention

Now, in general, it is required to improve usability, in treatment, of a treatment apparatus such as the above-described delivery catheter. What is therefore desired is a proposal that makes it possible to improve usability in such treatment.

The invention has been made in view of such circumstances, and it is an object of the invention to provide a treatment apparatus that makes it possible to improve usability in treatment.

A treatment apparatus of the invention includes a catheter that includes a delivery shaft. The delivery shaft extends in an axial direction and is configured to hold, in a tip end region, a stent graft having a reduced diameter. The delivery shaft includes a tube-shaped member and a plurality of core members. The tube-shaped member includes one or a plurality of lumens provided in the axial direction. The plurality of core members extend in the axial direction inside the tube-shaped member at least in the tip end region, and have plastic deformability. The plurality of core members are disposed substantially isotropically in an outer circumferential direction of the lumen inside the tube-shaped member.

In the treatment apparatus of the invention, the plurality of core members having the plastic deformability are disposed substantially isotropically in the outer circumferential direction of the lumen inside the tube-shaped member, at least in the tip end region of the delivery shaft of the catheter. This leads to the following, for example, upon treatment that places the stent graft having the reduced diameter in an affected area in a greatly-curved artery, by means of the catheter holding the stent graft. That is, even when reforming (shaping) of the plurality of core members having plastic deformability is performed in advance in accordance with a curved shape of the artery, it is more difficult for the tube-shaped member to kink. As a result, it is more difficult for the shape of the lumen located on inner side of the core members to be deformed (it is more difficult for the lumen to be crushed).

In the treatment apparatus of the invention, the delivery shaft further includes a covering layer that covers the lumen. The covering layer and at least one of the plurality of core members are in contact with each other. In this case, even when the reforming of the plurality of core members is performed in advance as described above, it is further more difficult for the tube-shaped member to kink. It is therefore further more difficult for the shape of the lumen to be deformed. As a result, usability in treatment is further improved.

Moreover, it is desirable that the plurality of core members have respective diameters that are about equal to or greater than a diameter of the lumen. Also in this case, even when the reforming of the plurality of core members is performed in advance as described above, it is further more difficult for the tube-shaped member to kink. It is therefore further more difficult for the shape of the lumen to be deformed. As a result, usability in treatment is further improved.

Further, it is desirable that the delivery shaft further include a reinforcing layer. The reinforcing layer is provided on an outer circumferential surface of the tube-shaped member in a region on base end side of the tip end region, and has one or a plurality of slits. It is also desirable that the slit be provided in a circumferential direction of the reinforcing layer or be provided in spiral on the reinforcing layer. The provision of the above-described slit in the enhancing layer enhances resistance against pulling force in the axial direction of the delivery shaft, while making it easier for the delivery shaft to be curved. As a result, usability in treatment is further improved.

It is to be noted that, regarding the number of the lumen provided and the number of core members provided, an example case may be mentioned that the number of the lumen provided is one, and the number of the core members provided is four.

Moreover, the treatment apparatus of the invention may further include, for example, the stent graft and a cover in addition to the catheter. The cover holds, in the tip end region, the stent graft having the reduced diameter.

According to the treatment apparatus of the invention, the plurality of core members having the plastic deformability are disposed substantially isotropically in the outer circumferential direction of the lumen inside the tube-shaped member, at least in the tip end region of the delivery shaft of the catheter. Therefore, upon treatment that places the stent graft in an affected area in an artery, for example, even when reforming of the core members is performed in advance in accordance with a curved shape of the artery, it is more difficult for the shape of the lumen located on inner side of the core members to be deformed. Hence, it is possible to improve usability in treatment by performing the treatment by means of the catheter described above.

### Brief Description of Drawings

[FIG. 1] FIG. 1 is a schematic diagram illustrating an example of an outline configuration of a treatment apparatus according to an embodiment of the invention.
[FIG. 2] FIG. 2 is a schematic diagram illustrating an example of a detailed configuration of inside of a cover illustrated in FIG. 1, etc.
[FIG. 3] FIG. 3 is a schematic cross-sectional view of an example of a detailed configuration of a delivery shaft of a catheter illustrated in FIG. 1.
[FIG. 4] FIG. 4 is a schematic diagram illustrating an example of a cross-sectional configuration of a tip end region of the delivery shaft illustrated in FIG. 3.
[FIG. 5] FIG. 5 is a schematic perspective view of an example of a detailed configuration of a reinforcing layer illustrated in FIG. 3.
[FIG. 6] FIG. 6 is a schematic diagram illustrating an example of a method of using the treatment apparatus illustrated in FIG. 1.
[FIG. 7] FIG. 7 is a schematic diagram illustrating an example of a method of placing a stent graft illustrated in FIG. 1.
[FIG. 8] FIG. 8 is a schematic diagram illustrating a cross-sectional configuration of a tip end region of a delivery shaft according to a comparative example. Modes for Carrying Out the Invention

Embodiments of the invention are described in detail below with reference to the drawings. It is to be noted that the description is given in the following order.
1. Embodiment (an example in which four core members are disposed isotropically in an outer circumferential direction of one lumen)
2. Modification Examples

### [1. Embodiment]

### [Outline Configuration]

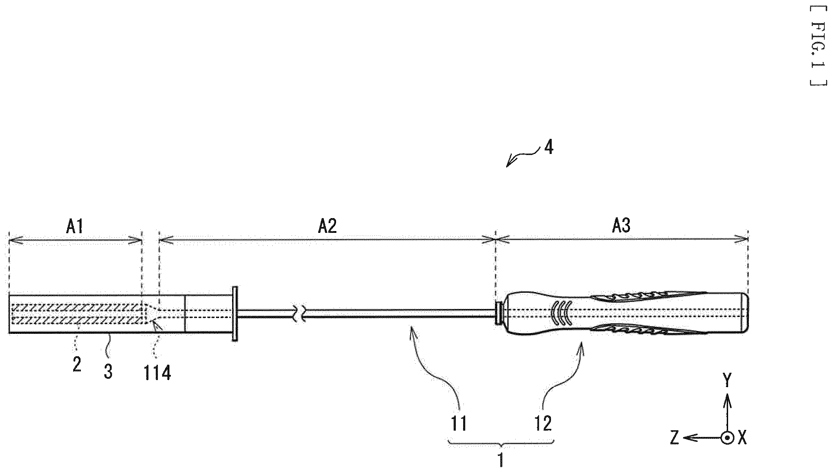

FIG. 1 is a schematic side view (a Y-Z side view) of an example of an outline configuration of a treatment apparatus (a treatment apparatus 4) according to an embodiment of the invention. The treatment apparatus 4 is an apparatus that is to be used, for example, upon treatment for arterial dissection, etc. utilizing the OSG method. In this example, the treatment apparatus 4 includes a catheter 1 (a delivery catheter), a stent graft 2, and a cover 3, as illustrated in FIG. 1. It is to be noted that the stent graft 2 is to be placed, by means of the catheter 1, at a site to be treated (for example, inside a blood vessel such as an artery), for example, upon the foregoing treatment, as will be described later in detail.

### [Catheter 1]

The catheter 1 is a medical device to be used upon delivery of the stent graft 2 to the foregoing site to be treated of a patient. The catheter 1 includes a delivery shaft 11 and a handle 12 (a holding part or a grip), as illustrated in FIG. 1.

The delivery shaft 11 includes a flexible pipe-shaped structure (pipe-shaped member), and has a shape extending (stretching) in a Z-axis direction. The Z-axis direction is an axial direction (a longitudinal direction) of the delivery shaft 11. The delivery shaft 11 includes a tip end region A1, an intermediate region A2, and a base end region A3 (an in-handle region) in this order in the axial direction (the Z-axis direction) from tip end side toward base end side, as illustrated in FIG. 1.

In the tip end region A1 of these regions, the later-described stent graft 2 having a reduced diameter is to be held inside the later-described cover 3 upon the foregoing treatment, as illustrated in FIG. 1. Further, the base end region A3 corresponds to a part contained inside the handle 12, as illustrated in FIG. 1. The intermediate region A2 is a region located between the tip end region A1 and the base end region A3, as illustrated in FIG. 1.

Moreover, an increased diameter part 114 (a flared part) is provided near a border between the tip end region A1 and the intermediate region A2, as illustrated in FIG. 1. The increased diameter part 114 has a funnel-like shape that flares on tip end region A1 side. Provision of the above-described increased diameter part 114 allows the base end side (the intermediate region A2 side) of the stent graft 2 to be fixed and thereby prevents the stent graft 2 from shifting and moving toward the base end side, for example, as illustrated in FIG. 1.

A length (a total length) of the above-described delivery shaft 11 in the axial direction is, for example, from about 300 mm to about 900 mm, preferably, from about 400 mm to about 800 mm, and further preferably, from about 450 mm to about 600 mm. One preferred example thereof is 570 mm. Further, a length of the tip end region A1 is set appropriately in accordance with a length of the stent graft 2 to be mounted. The length of the tip end region A1 is, for example, from about 20 mm to about 200 mm, preferably, from about 25 mm to about 150 mm, and further preferably, from about 30 mm to about 120 mm. A length of the intermediate region A2 is, for example, from about 200 mm to about 600 mm, preferably, from about 250 mm to about 550 mm, and further preferably, from about 300 mm to about 500 mm. One preferred example thereof is 350 mm.

Moreover, an outer diameter of the tip end region A1 is, for example, from 1.0 mm to about 8.0 mm, and preferably, from about 1.5 mm to about 6.0 mm. One preferred example thereof is 3.6 mm. An outer diameter of the intermediate region A2 is, for example, from about 2 mm to about 10 mm, and preferably, from about 3 mm to about 8 mm. One preferred example thereof is 5.0 mm. An outer diameter of the foregoing increased diameter part 114 is set appropriately in accordance with the diameter of the stent graft 2 having the reduced diameter. The outer diameter of the foregoing increased diameter part 114 is, for example, from about 3 mm to about 15 mm, and preferably, from about 5 mm to about 10 mm. One preferred example thereof is 8 mm.

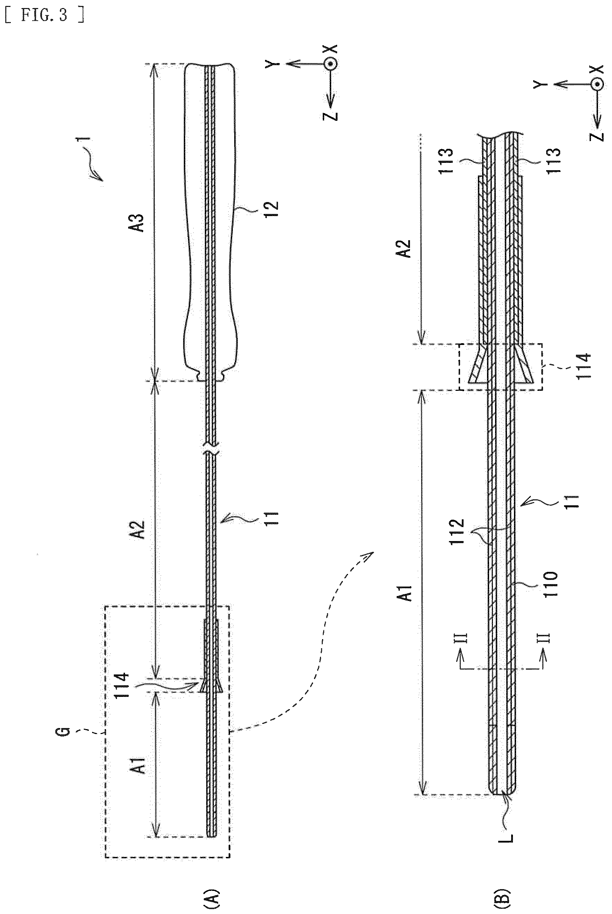

It is to be noted that an example of a detailed configuration of the above-described delivery shaft 11 will be described later (FIGs. 3 to 5).

The handle 12 is attached to a base end part (the base end region A3) of the delivery shaft 11, as illustrated in FIG. 1. The handle 12 is a part to be grabbed (held) by an operator (a doctor) using the catheter 1. The handle 12 has a shape that extends in its axial direction (the Z-axis direction).

A length of the handle 12 in the axial direction is, for example, from about 50 mm to about 200 mm, preferably, from about 60 mm to about 180 mm, and more preferably, from about 80 mm to about 150 mm. One preferred example thereof is 130 mm. Further, an outer diameter of the handle 12 is, for example, from about 3 mm to about 30 mm, and preferably, from about 5 mm to about 25 mm. One preferred example thereof is 20 mm. It is to be noted that the above-described handle 12 includes, for example, a synthetic resin such as polycarbonate or acrylonitrile butadiene styrene copolymer (ABS).

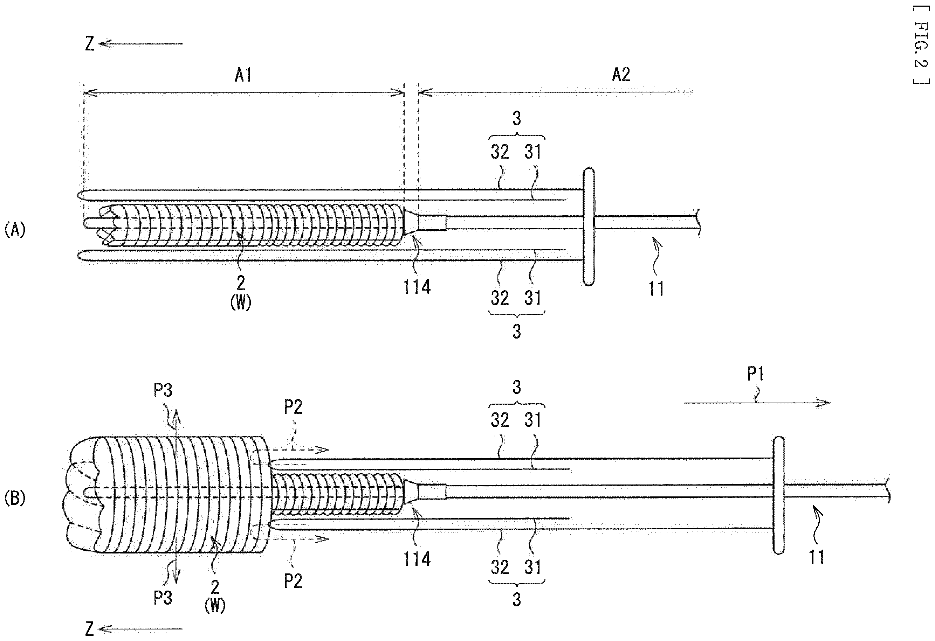

FIG. 2 is a schematic side view (a Y-Z side view) of an example of a detailed configuration of inside of the cover 3, etc. illustrated in FIG. 1. Specifically, Part (A) of FIG. 2 is a schematic side view of an example of a detailed configuration of the cover 3, the stent graft 2 inside the cover 3, the tip end region A1 of the delivery shaft 11 inside the cover 3, etc. Further, Part (B) of FIG. 2 is a schematic side view of an example of a state of an operation to be performed upon expansion of the stent graft 2 of the example of the configuration illustrated in Part (A) of FIG. 2.

### [Stent Graft 2]

As illustrated in Part (A) of FIG. 2 and Part (B) of FIG. 2, the stent graft 2 has a tubular (a cylindrical) structure that extends in its axial direction (the Z-axis direction). The stent graft 2 includes, for example, an unillustrated stent and an unillustrated graft. Further, the stent graft 2 has a self-expanding structure that is allowed to be maintained in a state having a reduced diameter. It is to be noted that the length of the stent graft 2 in the axial direction is, for example, from about 2 cm to about 30 cm. Further, an outer diameter of the expanded stent graft 2 is, for example, from about 6 mm to about 46 mm.

The foregoing stent includes one or a plurality of wire members W (wires). In this example, the foregoing stent has a tubular (a cylindrical) structure. Specifically, for example, the tubular structure includes a mesh structure, and such a tubular mesh structure is provided by weaving the wire member W in a predetermined pattern. It is to be noted that examples of the patterns of weaving may include plane weave, twill weave, stockinet, etc. Further, the tubular mesh structure may be provided by disposing one or more wire members W that are bent in a zigzag form and processed into a cylindrical shape.

It is to be noted that a metal wire member may be preferable as a material of the wire member W. In particular, a shape-memory alloy may be preferably employed. The shape-memory alloy is provided with a shape-memory effect, superelasticity, etc. by a thermal process. However, stainless steel, tantalum (Ta), titanium (Ti), platinum (Pt), gold (Au), tungsten (W), etc. may be used as the material of the wire member W, depending on the application. As the foregoing shape-memory alloy, for example, an alloy of nickel (Ni) and Ti, an alloy of copper (Cu), zinc (Zn), and X (X is aluminum (Al), iron (Fe), etc.), an alloy of Ni, Ti, and X (X is Fe, Cu, vanadium (V), cobalt (Co), etc.), etc. are preferable for use. It is to be noted that, for example, a synthetic resin, etc. may be used as the foregoing wire member W. Further, a metal wire member having a surface covered with Au, Pt, etc. by a method such as plating may be also used as the wire member W. Further, a complex wire member in which a core member is covered with an alloy may be used as the wire member W. The core member includes a material, such as Au or Pt, that does not transmit radiation.

Meanwhile, the foregoing graft has a tubular (cylindrical) shape, and is so disposed as to cover (coat) at least part of the stent. Specifically, for example, the graft is so disposed as to cover outer circumference side of the stent (the wire member W), or the graft is so disposed as to cover both the outer circumference side and inner circumference side of the stent (the wire member W).

Moreover, the graft is coupled to the stent, for example, by a method such as sewing, joining, or welding. In this case, the graft covers the stent and is coupled to the stent so as not to influence stretching and contraction of the stent. It is to be noted that a part at which the above-described graft and the stent are coupled to each other is provided appropriately, for example, at a location such as at both ends of the stent or in the middle of the stent.

Examples of a material that may be used as the graft described above include: a thermoplastic resin that is formed in to a tubular shape by a molding method such as extrusion molding or blow molding; a tubular knitted or woven material including a fiber of thermoplastic resin or a super-fine metal wire; a tubular non-woven fabric including a thermoplastic resin or super-fine metal; a tubular flexible resin sheet or a tubular porous sheet; a structure derived from a resin that has been dissolved in a solvent and is formed into a thin tubular shape by electrospinning; etc.

Here, as the foregoing knitted or woven material, a publicly-known material knitted or woven in plane weave, twill weave, etc. may be used. Further, a knitted or woven material with a crimp such as that subjected to a crimping process may also be used. It is to be noted that, in particular, the tubular knitted or woven material of the fiber of the thermoplastic resin may be preferable, and the tubular material woven in twill weave of the fiber of the thermoplastic resin may be more preferable among the foregoing materials. A reason for this is that such a material is superior in strength, pore rate, productivity, etc.

Moreover, as the foregoing thermoplastic resin, for example, a resin having durability and causing a small tissue reaction, etc. may be used. Examples of such a resin include: polyolefin such as polyethylene, polypropylene, or an ethylene-α-olefin copolymer; polyester such as polyamide, polyurethane, polyethylene terephthalate, polybutylene terephthalate, polycyclohexane terephthalate, or polyethylene-2, 6-naphthalate; fluororesin such as polyethylene fluoride or polypropylene fluoride; etc. It is to be noted that, among the foregoing materials, the polyester such as polyethylene terephthalate or the fluororesin such as polyethylene fluoride or polypropylene fluoride that are chemically stable, have great durability, and cause a small tissue reaction may be preferably used in particular.

### [Cover 3]

The cover 3 is a member that holds the stent graft 2 having the reduced diameter in the tip end region A1 of the delivery shaft 11, as described above. In this example, the cover 3 includes a soft cover. Covering the stent graft 2 having the reduced diameter with the soft cover ensures holding of the stent graft 2. Moreover, softness of the cover 3 makes it possible for the cover 3 to more easily follow variation in shape of the tip end region A1 of the delivery shaft 11, compared with a rigid tubular member (a sheath).

Moreover, as illustrated in Part (A) of FIG. 2 and Part (B) of FIG. 2, the cover 3 includes a bilayer structure (a double structure) having an inner layer part 31 and an outer layer part 32 in this example. Specifically, the cover 3 is provided by folding a tubular tube material to inside thereof. The folded part corresponds to the inner layer part 31. Moreover, as illustrated in Part (A) of FIG. 2, the tip end of the stent graft 2 having the reduced diameter is located on folded part side of the cover 3, and the stent graft 2 is thus disposed between the folded part and a base end of the inner layer part 31.

Such a configuration allows for an operation to be performed upon the expansion of the stent graft 2, for example, as illustrated in Part (B) of FIG. 2. That is, first, a base end of the outer layer part 32 is pulled by the operator of the catheter 1 in a direction toward the base end of the delivery shaft 11 (a direction away from the foregoing folded part) (see an arrow P1). Accordingly, the inner layer part 31 is peeled off from the outer circumference of the stent graft 2 to be removed from the stent graft 2 (see an arrow P2). As a result, the stent graft 2 is expanded (deployed) gradually from its tip end side (see an arrow P3). It is to be noted that, upon the gradual expansion, the force to pull the cover 3 derives from frictional resistance between an outer surface of the inner layer part 31 and an inner surface of the outer layer part 32. Therefore, the force to pull the cover 3 is not influenced by the expanding force of the stent graft 2 having the reduced diameter. Accordingly, it is possible to ensure deployment of the stent graft 2 also in a case where the stent graft 2 has great expanding force.

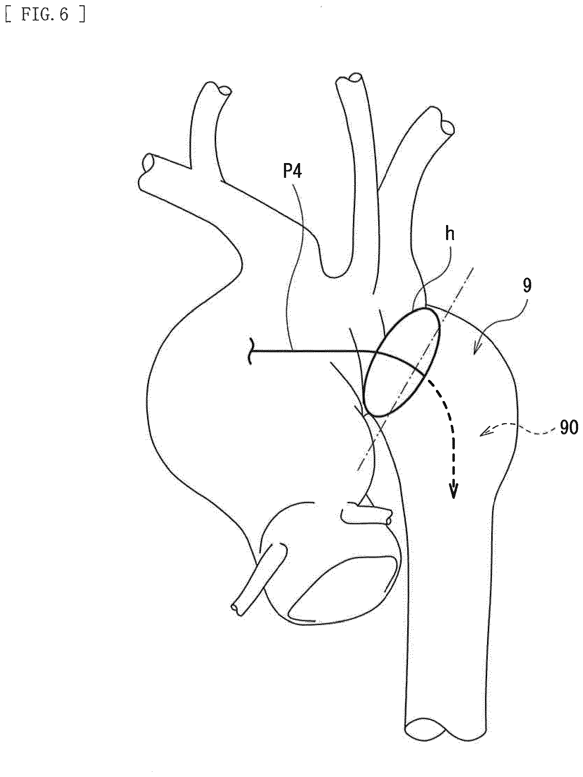

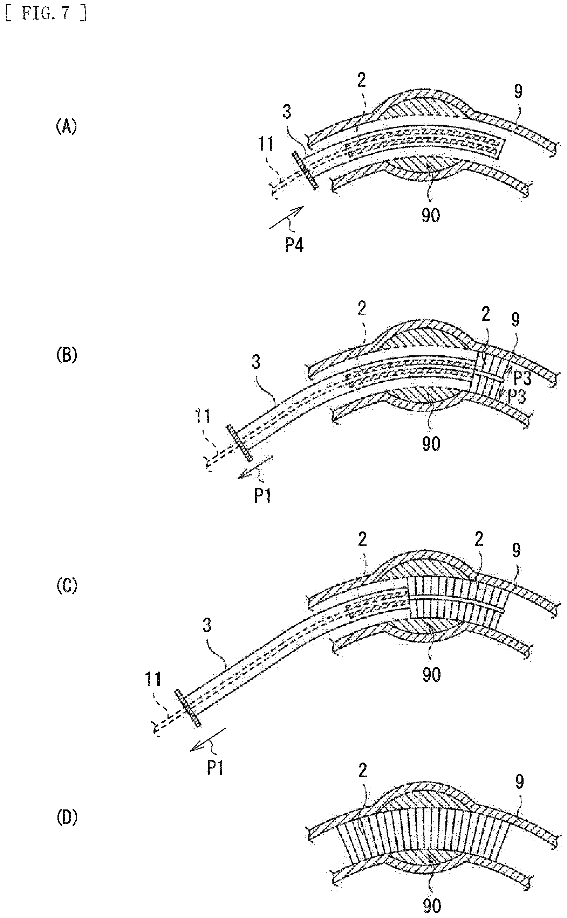

It is to be noted that the operation to be performed upon such expansion of the stent graft 2 (a method of placing the stent graft 2 upon treatment) will be described later in greater detail (FIGs. 6 and 7).

### [Detailed Configuration]

Next, a description is given, with reference to FIGs. 3 to 5, of an example of a detailed configuration of the delivery shaft 11 of the catheter 1 illustrated in FIGs. 1 and 2.

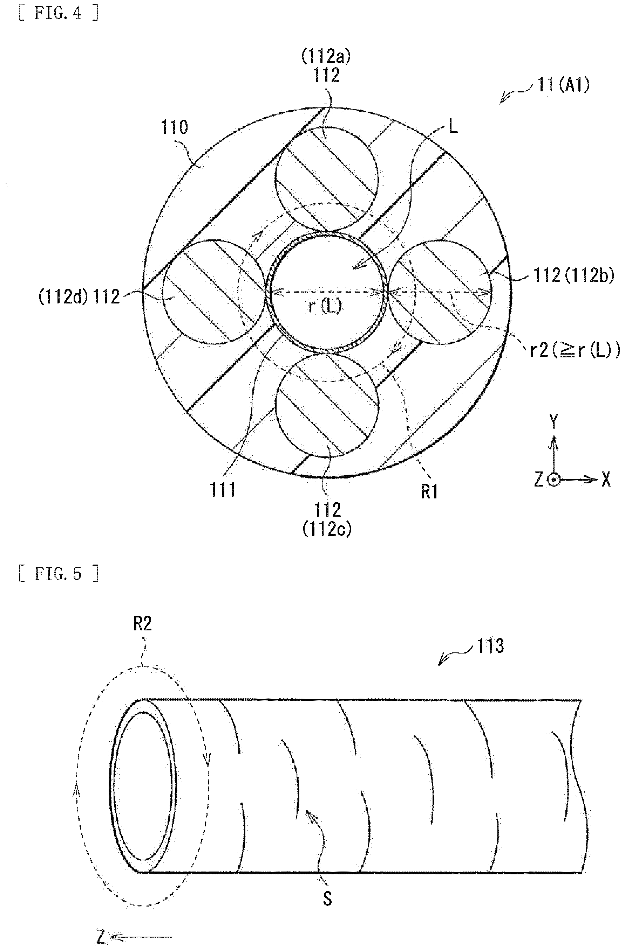

FIG. 3 is a schematic cross-sectional view (a Y-Z cross-sectional view) of an example of the detailed configuration of the delivery shaft 11. It is to be noted that Part (B) of FIG. 3 illustrates, in an enlarged manner, a part indicated by a symbol G in Part (A) of FIG. 3 (a part around the tip end region A1). Further, FIG. 4 schematically illustrates an example of a cross-sectional configuration (an example of an X-Y cross-sectional configuration) of a part, of the delivery shaft 11, taken along a line II-II in Part (B) of FIG. 3. FIG. 5 is a schematic perspective view of an example of a detailed configuration of a reinforcing layer (a reinforcing layer 113 which will be described later) illustrated in FIG. 3.

First, as illustrated in FIGs. 3 and 4, the delivery shaft 11 includes, in this example, a tube-shaped member 110, a covering layer 111, a plurality of core members 112 (four core members 112a, 12b, 12c, and 12d, in this example), the reinforcing layer 113, and the increased diameter part 114 described above.

### [Tube-shaped Member 110]

The tube-shaped member 110 includes a single lumen L (has a single lumen structure) provided in its axial direction (the Z-axis direction). The lumen L serves as an insertion path of a guide wire, as will be described later. Securing of such an insertion path makes it possible to insert the guide wire into the lumen L before or after the insertion or placement of the stent graft 2. It is to be noted that a diameter of the above-described lumen L is, for example, from about 0.9 mm to about 2.5 mm. One preferred example thereof is 1.12 mm.

The tube-shaped member 110 described above may include, for example, a synthetic resin such as polyolefin, polyamide, polyetherpolyamide, polyurethane, nylon, or polyether block amide.

### [Covering Layer 111]

The covering layer 111 is a layer that covers the entire circumference of the lumen L, as illustrated in FIG. 4. The covering layer 111 has a thickness, for example, from about 0.03 mm to about 0.08 mm. The covering layer 111 also serves as a sliding material to be used upon insertion of the above-described guide wire, etc. into the lumen L.

The covering layer 111 described above includes, for example, a resin material (a fluorine-based resin such as PFA or PTFE, etc.).

### [Core Member 112]

The core member 112 is a member having plastic deformability. As illustrated in FIGs. 3 and 4, the core member 112 extends in the axial direction (the Z-axis direction) inside the tube-shaped member 110 at least in the tip end region A1 of the delivery shaft 11. A plurality of such core members 112 (four core members, i.e., core members 112a, 112b, 112c, and 112d, in this example) are disposed side by side inside the tube-shaped member 110, as illustrated in FIG. 4. Further, in this example, the core members 112 (112a to 112d) are each a columnar member as illustrated in FIG. 4, and are provided for substantially the entire length of the delivery shaft 11 (in each of the tip end region A1, the intermediate region A2, and the base end region A3). This secures sufficient rigidity of the delivery shaft 11 and improves operability of the catheter 1.

It is to be noted that the "plastic deformability" as used herein refers to a property that allows, when the tip end region A1 of the delivery shaft 11 is deformed by force of one's hand, one's finger, etc., the deformed shape to be maintained. It is also required to maintain the deformed shape against restoring force (elastic force that attempts to return to a straight shape) of the stent graft 2 that is deformed together with the tip end region A1.

Here, in the present embodiment, the plurality of core members 112 (112a to 112d) are disposed substantially isotropically (desirably, isotropically) in an outer circumferential direction R1 of the lumen L inside the tube-shaped member 110, for example, as illustrated in FIG. 4. Specifically, in this example, the four core members 112a to 112d are disposed in the outer circumferential direction R1 at 90-degree intervals, as illustrated in FIG. 4. In detail, the core member 112a is disposed on upper side (+Y-axis side) of the lumen L, the core member 112b is disposed on right side (+X-axis side) of the lumen L, the core member 112c is disposed on lower side (-Y-axis side) of the lumen L, and the core member 112d is disposed on left side (-X-axis side) of the lumen L. It is to be noted that "substantially isotropically" described above refers to having a positional deviation within, for example, about ±10% on the basis of an "isotropic" arrangement as a reference.

Moreover, in the present embodiment, the covering layer 111 (the lumen L) and at least one (all of the core members 112a to 112d, in this example) of the plurality of core members 112 (112a to 112d) are in contact with each other, for example, as illustrated in FIG. 4.

Moreover, in the present embodiment, diameters (outer diameters) r2 of the respective plurality of core members 112 (112a to 112d) are about equal to or greater than a diameter r(L) of the lumen L, for example, as illustrated in FIG. 4. In other words, the diameter r2 is substantially equal to the diameter r(L) (r2 ≈r(L)), or the diameter r2 is greater than the diameter r(L) (r2 >r(L)). It is also possible that the diameter r2 is equal to the diameter r(L) (r2 =r(L)). It is to be noted that "about equal to or greater than" described above refers to having an error within, for example, about 10% on the basis of an "equal" value as a reference (refers to that the diameter r2 has a value that is 90% or greater of that of the diameter r(L)). Further, the diameter r2 of the core member 112 is, for example, from about 0.5 mm to about 3 mm, and preferably, from about 1 mm to about 2 mm. One preferred example thereof is 1.15 mm.

The above-described core material 112 includes a metal material having the foregoing plastic deformability. Examples of the metal material includes metal and an alloy such as stainless steel (SUS), titanium, a titanium alloy, an alloy of cobalt and chromium, an alloy of nickel and chromium, an alloy of chromium and molybdenum, aluminum, an aluminum alloy, a magnesium alloy, a tantalum alloy, a zirconium alloy, gold, platinum, copper, or an alloy of gold, silver, and palladium.

### [Reinforcing Layer 113]

The reinforcing layer 113 is a member that secures rigidity (reinforcing rigidity) of a region, of the delivery shaft 11, on the base end side (the intermediate region A2 and the base end region A3, in this example). For example, as illustrated in Part (B) of FIG. 3, the reinforcing layer 113 is so disposed as to cover the outer circumferential surface of the tube-shaped member 110 in a region (the intermediate region A2 and the base end region A3, in this example), of the delivery shaft 11, on the base end side of the tip end region A1.

Moreover, for example, as illustrated in FIG. 5, the reinforcing layer 113 have one or a plurality of (a plurality of, in this example) slits S provided in its circumferential direction R2.

A thickness of the above-described reinforcing layer 113 is, for example, from about 0.1 mm to about 0.3 mm. Further, the reinforcing layer 113 includes, for example, a material such as stainless steel (SUS). In one example, the reinforcing layer 113 includes a SUS pipe. It is to be noted that the outer circumferential surface of the reinforcing layer 113 including the SUS pipe described above may be further covered with, for example, a resin tube including polyether block amide, etc.

### [Operation, Workings, and Effects]

### [A. Basic Operation]

The treatment apparatus 4 is used, for example, upon treatment for arterial dissection, etc. Specifically, by an operator operating the catheter 1, the stent graft 2 having a reduced diameter is delivered to a site, of a patient, to be treated (for example, inside a blood vessel such as artery) and is placed at the site after being expanded. Thus placing the stent graft 2 at the site to be treated makes it possible to expand and retain a lumen of a blood vessel while suppressing flowing of blood into a ruptured part of an inner wall of a blood vessel. In particular, the stent graft 2 is also used, for example, upon treatment utilizing the OSG method which is one of treatment methods for arterial dissection in a thoracic aorta, etc.

Here, a description is given of an outline of the treatment method for arterial dissection (aneurysm), etc. utilizing the OSG method, with reference to FIGs. 6 and 7.

FIG. 6 is a schematic diagram illustrating an example of a method of using the treatment apparatus 4 upon the foregoing treatment. Further, FIG. 7 (Part (A) of FIG. 7 to Part (D) of FIG. 7 is a schematic diagram illustrating an example of a method of placing the stent graft 2 upon the foregoing treatment. It is to be noted that a description is given here referring to an example case where an artery 9 (a thoracic aorta) that is a blood vessel to be treated is a descending aorta. Further, an aneurysm in the artery 9 to be treated is illustrated as an aneurysm 90 in FIGs. 6 and 7.

For example, as illustrated in FIG. 6, in the OSG method, first, the stent graft 2 having a reduced diameter is inserted from an opening "h" by means of the treatment apparatus 4 (see an arrow P4), after a thoracic part of a patient is opened. The opening "h" derives from incision of part of the artery 9. The treatment apparatus 4 has the configuration illustrated in FIGs. 1 to 5. Specifically, the stent graft 2 is inserted from the tip end side (the cover 3 side) of the delivery shaft 11 of the catheter 1. On this occasion, the stent graft 2 having the reduced diameter is held inside the cover 3 in the tip end region A1 of the delivery shaft 11 of the catheter 1, for example, as illustrated in FIGs. 1 and 2.

Here, in an example case where the artery 9 to be treated is in an arterial dissection state, an artery lumen (a true lumen) that is an appropriate blood flow path and a new lumen (a false lumen) derived from rupture of an inner wall of the blood vessel are present inside the artery 9. In such a case, the catheter 1 is inserted along the guide wire described above to thereby avoid erroneous insertion of the catheter 1 into the false lumen. In other words, the catheter 1 is inserted while inserting such a guide wire in the lumen L of the catheter 1.

Specifically, first, the guide wire is inserted from a base of the patient's leg (groin) into the artery 9 and led to outside from the opening "h" via inside of the artery 9. On this occasion, the guide wire is inserted from terminal side into the artery 9. This prevents the guide wire from being erroneously inserted into the false lumen and ensures insertion of the guide wire into the true lumen. It is to be noted that a length of the above-described guide wire is, for example, from about 50 cm to about 450 cm, and an outer diameter thereof is, for example, from about 0.2 mm to about 1.0 mm.

Thereafter, the catheter 1 is inserted from the opening "h" as an entering opening into the artery 9 along the guide wire (see the arrow P4). The guide wire is inserted into the true lumen for certain, as described above. Therefore, the insertion of the catheter 1 into the artery 9 along the guide wire ensures insertion of the catheter 1 into the true lumen in turn. In other words, the catheter 1 is prevented from being inserted into the false lumen also in the case of the arterial dissection state.

Thereafter, for example, as illustrated in Part (A) of FIG. 7, the stent graft 2 is caused to reach a site located farther than the site, of the artery 9, to be treated (a part near a location provided with the aneurysm 90), by means of the treatment apparatus 4 (see the arrow P4).

Thereafter, for example, as illustrated in Part (B) of FIG. 7 and Part (C) of FIG. 7, an operation of increasing the diameter of the stent graft 2 (deployment of the stent graft 2) is performed by utilizing self-expanding force of the stent graft 2. Specifically, first, the base end of the outer layer part 32 of the cover 3 is pulled in the direction toward the base end of the delivery shaft 11 by the operator of the catheter 1 (see the arrow P1). Accordingly, the inner layer part 31 is peeled off from the outer circumference of the stent graft 2 to be removed from the stent graft 2 (see the arrow P2 in Part (B) of FIG. 2 described above). As a result, the stent graft 2 expands gradually from its tip end side (see the arrow P3).

Accordingly, for example, as illustrated in Part (D) of FIG. 7, the stent graft 2 is fixed to the inner wall of the artery 9. As a result, the lumen of the artery 9 near the location provided with the aneurysm 90 is expanded and retained. Thereafter, an anastomosis is made between the base end side of the stent graft 2 and the artery 9 (the blood vessel of the patient) by suturing. It is to be noted that, further, another anastomosis may be made between the foregoing anastomosis part and another artificial blood vessel different from the stent graft 2 on an as-needed basis.

An inner circumference of the aneurysm 90 is thus covered with the stent graft 2. This allows blood to flow through inside of the stent graft 2, and the flow of the blood into the ruptured part of the inner wall of the blood vessel is blocked. As a result, the aneurysm 90 is not influenced by a blood pressure, etc. Hence, it is possible to prevent an increase in diameter of the aneurysm 90 and rupture of the blood vessel at the aneurysm 90. It is also possible to maintain a blood flow at the aneurysm 90.

Moreover, the foregoing treatment method utilizing the OSG method has the following advantages in particular, compared with a treatment method (an existing treatment method) that inserts a catheter from a base of the patient's leg (groin) to deliver a stent graft to a part to be treated. That is, it is possible to perform a procedure on a part having an important branch (for example, an aortic arch) which has caused extreme difficulty in procedure by the existing treatment method, which is advantageous. Moreover, when compared with a method that replaces a removed diseased part by an artificial blood vessel, and makes an anastomosis on each side of the artificial blood vessel, fixing with the stent graft 2 is performed instead of suturing of the descending aorta (instead of making an anastomosis on terminal side). In other words, the OSG method omits making the anastomosis between the tip end side of the stent graft 2 and the descending aorta. It is therefore easier to make an anastomosis. This reduces time for operation (extracorporeal circulation time), and in addition, avoids opening of a left thoracic part or a great thoracic incision that is necessary for the suturing of the descending aorta. Therefore, operative invasion on the patient is reduced (a burden on the patient is reduced upon the treatment). Moreover, the OSG method makes it possible to set a transplantation range of the artificial blood vessel to a great range. This makes it possible to perform a surgical procedure for a complication near the treated part, which is also advantageous. In addition, the stent graft applied to the OSG method is not introduced from a groin, unlike the foregoing existing treatment method. This eliminates the necessity to cause the stent graft to pass through a fine blood vessel. It is therefore possible for the stent graft to have an outer diameter that is great (thick) to some extent even in a state of having a reduced diameter.

### [B. Workings and Effects of Delivery Shaft 11]

Now, for example, the following issues may arise upon treatment that places a stent graft in an affected area of a greatly-curved artery by means of a typical treatment apparatus (a catheter holding a stent graft having a reduced diameter). The greatly-curved artery is, for example, the artery 9 (a descending aorta) illustrated in FIGs. 6 and 7. That is, it is difficult in technique to place, by means of the treatment apparatus, the stent graft at an appropriate position in the affected area, inside the greatly-curved artery, where the blood flow is blocked and which is reduced in size.

Specifically, a tip end region of a delivery shaft of a catheter typically has a straight shape. Accordingly, a stent graft having a reduced diameter and is held in the tip end region is also made straight. Moreover, the delivery shaft typically includes an elastic material such as a resin. Therefore, for example, even when a curving load is applied to the stent graft lend in the tip end region of the delivery shaft, the straight shape is restored immediately due to elasticity of the tip end region and of the stent graft. Accordingly, in the case where the stent graft is inserted in the greatly-curved artery, the stent graft held in the tip region of the delivery shaft is not allowed to be held in a shape in accordance with the curved shape of the artery. Therefore, the stent graft is delivered, deployed (expanded), and placed while kept being straight.

As a result, the deployed stent graft is not allowed to be attached sufficiently to the inner wall of the greatly-curved artery, which leads to the following. That is, first, so-called "endoleak" occurs in which blood flows into a gap between the inner wall and the stent graft. This makes it difficult to maintain the lumen of the artery (to expand and retain the lumen of the blood vessel while securing the blood flow into the stent graft and blocking the blood flow into the ruptured part in the inner wall of the blood vessel). Further, it also becomes difficult to accurately dispose the straight stent graft (in particular, the end of the straight stent graft) at a position relative to a greatly-curved target site. This may lead to a failure in maintaining the lumen of the artery, or in blocking the blood flow into the aneurysm. This may also lead to occlusion of a useful blood vessel that is branched from the vicinity of the position at which the stent graft is placed. Further, the straight stent graft may kink inside the greatly-curved artery, which may lead to a failure in securing a blood flow.

To address this, the treatment apparatus 4 of the present embodiment is configured to allow an operator to perform the following operation upon the treatment that places the stent graft 2 in an affected area of a greatly-curved artery, for example, in the artery 9 (the descending aorta) illustrated in FIGs. 6 and 7. That is, reforming (shaping) of the core member 112 having plastic deformability and mounted in the delivery shaft 11 (the tube-shaped member 110) of catheter 1 is performed in advance in accordance with the curved shape of the artery described above. In other words, the tip end region A1 of the delivery shaft 11 that holds the stent graft 2 is shaped into a shape in accordance with the curved shape of the artery.

Accordingly, in the present embodiment, it is possible to maintain the stent graft 2 delivered to an affected area in the shape in accordance with the curved shape of the artery 9 (in the shape provided by the shaping), for example, as illustrated in Part (A) of FIG. 7 to Part (D) of FIG. 7 described above. This owes to the rigidity, of the core member 112 having the plastic deformability, which maintains the shape of the core member 112. As a result, it is possible to deploy the stent graft 2 having the reduced diameter in a state in accordance with the curved shape of the greatly-curved artery, and place the stent graft 2 in the shape in accordance with the curved shape of the artery 9. The stent graft 2 thus deployed (expanded) has preferred attachment characteristics with respect to the inner wall of the artery 9 and is accurately placed relative to the target site, for example, as illustrated in Part (D) of FIG. 7. Therefore, an aimed treatment effect (maintaining of a lumen of an artery, prevention of rupture of the aneurysm, etc. described above) is obtainable for certain.

### [Comparative Example]

In the technique of reforming the core member 112 having plastic deformability in advance in accordance with the curved shape of the artery 9 may have, however, the following issue depending on the configuration (the number, an arrangement, etc.) of the core members 112 inside the tube-shaped member 110. That is, the tube-shaped member 110 may kink depending on the configuration of the core members 112, which may deform the shape of the lumen L located on the inner side of the core members 112 (may crush the lumen L).

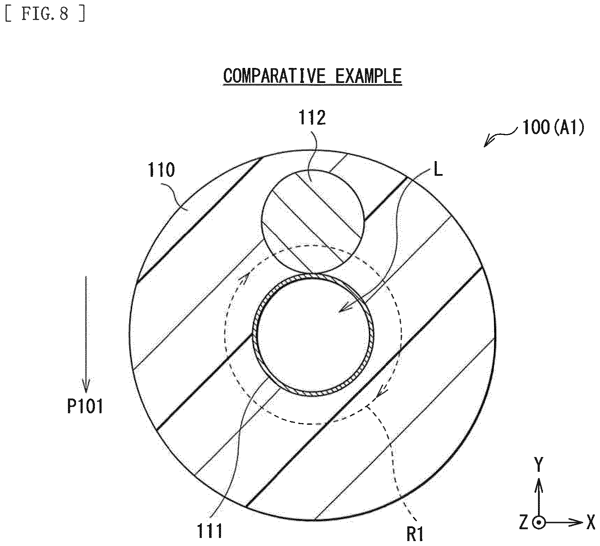

Specifically, for example, a delivery shaft 100 (the tip end region A1) according to a comparative example illustrated in FIG. 8 includes the core member 112 having the following configuration inside the tube-shaped member 110, unlike the delivery shaft 11 (the tip end region A1) of the present embodiment illustrated in FIG. 4. That is, the single core member 112 is anisotropically disposed in the outer circumferential direction R1 of the lumen L in the tube-shaped member 110 of the delivery shaft 100 (the arrangement of the core member 112 is imbalanced).

Accordingly, for example, in a case where an operation of reforming the core member 112 is repeatedly performed in a direction indicated by an arrow P101 in FIG. 8, etc. the following happens in the comparative example, unlike the present embodiment described below. That is, the kinking of the tube-shaped member 110 and accompanying deformation of the shape of the lumen L located on the inner side of the core member 112 (crushing of the lumen L) described above may make it impossible, for example, to insert the above-described guide wire into the lumen L. Hence, a sufficient treatment effect may not be obtainable, for example, in a case of performing treatment for arterial dissection (aneurysm), etc. by utilizing the OSG method by means of a catheter having the delivery shaft 100 of the comparative example. In other words, usability is decreased in the comparative example, for example, upon the treatment for arterial dissection (aneurysm), etc. utilizing the OSG method.

### [Present Embodiment]

In contrast, the catheter 1 of the treatment apparatus 4 of the present embodiment is configured as follows, unlike the above-described comparative example.

That is, first, the plurality of core members 112 (112a to 112d) are disposed substantially isotropically in the outer circumferential direction R1 of the lumen L inside the tube-shaped member 110 at least in the tip end region A1, for example, as the delivery shaft 11 illustrated in FIG. 4. This allows for the following, for example, when the treatment is performed that places the stent graft 2 in an affected area of a greatly-curved artery (such as the artery 9 illustrated in FIG. 6) by means of the catheter 1 holding the stent graft 2 having the reduced diameter in the tip end region A1 as described above.

That is, even when the reforming of the plurality of core members 112 having plastic deformability is performed in advance in accordance with the curved shape of the artery 9, etc. as described above, it is more difficult for the tube-shaped member 110 to kink, unlike the above-described comparative example. As a result, it is more difficult for the shape of the lumen L located on the inner side of the core members 112 to be deformed (it is more difficult for the lumen L to be crushed). Accordingly, it is possible to insert the above-described guide wire, for example, in the lumen L.

Moreover, in the present embodiment, the covering layer 111 (the lumen L) and at least one (all of the core members 112a to 112d, in this example) of the plurality of core members 112 (112a to 112d) are in contact with each other, for example, as illustrated in FIG. 4. Accordingly, even when the reforming of the plurality of core members 112 is performed in advance as described above, it is further more difficult for the tube-shaped member 110 to kink. It is therefore further more difficult for the shape of the lumen L to be deformed. As a result, usability in treatment is further improved.

Moreover, in the present embodiment, the diameters r2 of the respective plurality of core members 112 (112a to 112d) are about equal to or greater than the diameter r(L) of the lumen L, for example, as illustrated in FIG. 4. This leads to the following even when the reforming of the plurality of core members 112 is performed in advance as described above. That is, also in this point of view, it is further more difficult for the tube-shaped member 110 to kink. It is therefore further more difficult for the shape of the lumen L to be deformed. As a result, usability in treatment is further improved.

In addition, in the present embodiment, the reinforcing layer 113 is provided on the outer circumferential surface of the tube-shaped member 110 in the region (the intermediate region A2 and the base end region A3, in this example), of the delivery shaft 11, on the base end side of the tip end region A1, for example, as illustrated in Part (B) of FIG. 3. Moreover, the reinforcing layer 113 have one or the plurality of (the plurality of, in this example) slits S provided in its circumferential direction R2, for example, as illustrated in FIG. 5. Such provision of the slit S in the circumferential direction R2 of the reinforcing layer 113 enhances resistance against pulling force in the axial direction (the Z-axis direction) of the delivery shaft 11, while making it easier for the delivery shaft 11 to be curved. As a result, usability in treatment is further improved.

Specifically, in a case where compression force (force in the axial direction) is applied to the reinforcing layer 113, end surfaces of the respective slits S come into surface contact with each other. It is therefore more difficult for positional deviation to occur, which allows the shape of the reinforcing layer 113 to be maintained. Accordingly, even in a case where an operation of pulling the base end of the outer layer part 32 of the cover 3 in the direction toward the base end of the delivery shaft 11 is performed in order to increase the diameter of the stent graft 2, it is possible to prevent curving of the region, of the delivery shaft 11, on the base end side of the tip end region A1. It is to be noted that, in a case where the above-described region on the base end side of the tip end region A1 is curved, for example, an issue derived from the "endoleak" described above, etc. may possibly arise. Moreover, the reinforcing layer 113 is easily curved by force other than that in the axial direction. It is therefore possible to also perform reforming of a shape of the region, of the delivery shaft 11, on the base end side of the tip end region A1 on an as-needed basis.

In the present embodiment, the plurality of core members 112 having plastic deformability are disposed substantially isotropically in the outer circumferential direction R1 of the lumen L inside the tube-shaped member 110 at least in the tip end region A1 of the delivery shaft 11 of the catheter 1, as described above, which leads to the following. That is, upon the treatment of placing the stent graft 2 in an affected area such as the artery 9, it is possible to make it more difficult for the shape of the lumen L located on the inner side of the core member 112 to be deformed, for example, even when the reforming of the core member 112 is performed in advance in accordance with the curved shape of the artery 9, etc. Accordingly, it is possible to improve usability in treatment by performing the treatment by means of the catheter 1 described above.

### [2. Modification Examples]

Although the disclosure has been described above with reference to an embodiment, the disclosure is not limited to the embodiment but may be modified in a wide variety of ways.

For example, shapes, locations, sizes, numbers, materials, etc., of the respective members described in the foregoing embodiment are non-limiting, and may respectively be any other shape, location, size, number, material, etc. Specifically, for example, a tube-shaped member having a plurality of lumens (a multi-lumen structure) may be used. Moreover, the core member may be provided only in a partial region including the tip end region. Further, the number of the provided core members may be any number greater than one and other than four (for example, two, three, or greater than five). In addition, the substantially-isotropic arrangement configuration of the plurality of core members is not limited to that described in the foregoing embodiment, but may be any other arrangement configuration example. Moreover, the foregoing embodiment has been described referring to the example case where the one or the plurality of slits S are provided in the circumferential direction R2 of the reinforcing layer 113; however, this example is non-limiting. In other words, for example, one or a plurality of spiral slits (one or a plurality of slits provided in a manner winding around the reinforcing layer 113 in an oblique direction) may be provided on the reinforcing layer 113.

In addition, the diameter of the core member may be smaller than the diameter of the lumen in some cases. Moreover, the increased diameter part may not be provided near the border between the tip end region and the intermediate region of the delivery shaft in some cases.

Further, the foregoing embodiment has been described referring to the example case where the treatment apparatus includes the catheter, the stent graft, and the cover; however, this is non-limiting. In other words, for example, the treatment apparatus may include only the catheter, may include the catheter and the stent graft, or may include the catheter and the cover.

In addition, the foregoing embodiment has been described mainly referring to the example of the treatment apparatus (the catheter, the stent graft, etc.) to be applied to the treatment for a descending aorta; however, this is non-limiting. In other words, the treatment apparatus of the invention is also applicable to treatment for a blood vessel such as an artery other than a descending aorta (for example, an ascending aorta, an aortic arch, a thoracoabdominal aorta, an abdominal aorta, an iliac artery, a femoral artery, etc.).

## Claims

1. A treatment apparatus (4) comprising
a catheter (1) that includes a delivery shaft (11), the delivery shaft (11) extending in an axial direction (Z) and configured to hold, in a tip end region (A1), a stent graft (2) having a reduced diameter,
the delivery shaft (11) including
a tube-shaped member (110) that includes one or a plurality of lumens (L) provided in the axial direction (Z), and
a plurality of core members (112, 112a, 112b, 112c, 112d) that extend in the axial direction (Z) inside the tube-shaped member (110) at least in the tip end region (A1), and have plastic deformability, the plurality of core members (112, 112a, 112b, 112c, 112d) being disposed substantially isotropically in an outer circumferential direction (R1) of the lumen (L) inside the tube-shaped member (110); wherein
the delivery shaft (11) further includes a covering layer (111) that covers the lumen (L), and
the covering layer (111) and at least one of the plurality of core members (112, 112a, 112b, 112c, 112d) are in contact with each other.

2. The treatment apparatus (4) according to claim 1, wherein the plurality of core members (112, 112a, 112b, 112c, 112d) have respective diameters (r2) that are about equal to or greater than a diameter (r(L)) of the lumen (L).

3. The treatment apparatus (4) according to any one of claims 1 or 2, wherein
the delivery shaft (11) further includes a reinforcing layer (113), the reinforcing layer (113) being provided on an outer circumferential surface of the tube-shaped member (110) in a region (A2, A3) on base end side of the tip end region (A1), and having one or a plurality of slits (S), and
the slit (S) is provided in a circumferential direction (R2) of the reinforcing layer (113) or is provided in spiral on the reinforcing layer (113).

4. The treatment apparatus (4) according to any one of claims 1 to 3, wherein number of the lumen (L) provided is one, and number of the core members (112, 112a, 112b, 112c, 112d) provided is four.

5. The treatment apparatus (4) according to any one of claims 1 to 4, further comprising:
the stent graft (2); and
a cover (3) that holds, in the tip end region (A1), the stent graft (2) having the reduced diameter.

## Patentansprüche

1. Behandlungseinrichtung (4), umfassend
einen Katheter (1), der einen Abgabeschaft (11) einschließt, wobei sich der Abgabeschaft (11) in eine Axialrichtung (Z) erstreckt und konfiguriert ist, um, in einer Spitzenendregion (A1), ein Stenttransplantat (2), das einen reduzierten Durchmesser aufweist, zu halten,
wobei der Abgabeschaft (11) einschließt
ein röhrenförmiges Element (110), das ein oder eine Vielzahl von Lumen (L), die in der Axialrichtung (Z) bereitgestellt ist, einschließt und
eine Vielzahl von Kernelementen (112, 112a, 112b, 112c, 112d), die sich in der Axialrichtung (Z) im Inneren des röhrenförmigen Elements (110) mindestens in die Spitzenendregion (A1) erstreckt und eine plastische Verformbarkeit aufweist, wobei die Vielzahl von Kernelementen (112, 112a, 112b, 112c, 112d) in einer äußeren Umfangsrichtung (R1) des Lumens (L) im Inneren des röhrenförmigen Elements (110) im Wesentlichen isotrop angeordnet ist; wobei
der Abgabeschaft (11) ferner eine Umhüllungsschicht (111), die das Lumen (L) umhüllt, einschließt und
die Umhüllungsschicht (111) und mindestens eines der Vielzahl von Kernelementen (112, 112a, 112b, 112c, 112d) in Verbindung miteinander stehen.

2. Behandlungseinrichtung (4) nach Anspruch 1, wobei die Vielzahl von Kernelementen (112, 112a, 112b, 112c, 112d) jeweilige Durchmesser (r2), die etwa gleich oder größer als ein Durchmesser (r(L)) des Lumens (L) sind, aufweist.

3. Behandlungseinrichtung (4) nach einem der Ansprüche 1 oder 2, wobei der Abgabeschaft (11) ferner eine Verstärkungsschicht (113) einschließt, wobei die Verstärkungsschicht (113) an einer äußeren Umfangsoberfläche des röhrenförmigen Elements (110) in einer Region (A2, A3) an einer Basisendseite der Spitzenendregion (A1) bereitgestellt ist und einen oder eine Vielzahl von Schlitzen (S) aufweist, und
der Schlitz (S) in einer Umfangsrichtung (R2) der Verstärkungsschicht (113) bereitgestellt ist oder an der Verstärkungsschicht (113) spiralig bereitgestellt ist.

4. Behandlungseinrichtung (4) nach einem der Ansprüche 1 bis 3, wobei eine Anzahl des bereitgestellten Lumens (L) eins beträgt und die Anzahl der bereitgestellten Kernelemente (112, 112a, 112b, 112c, 112d) vier beträgt.

5. Behandlungseinrichtung (4) nach einem der Ansprüche 1 bis 4, ferner umfassend:
das Stenttransplantat (2); und
eine Umhüllung (3), die, in der Spitzenendregion (A1), das Stenttransplantat (2), das den reduzierten Durchmesser aufweist, hält.

## Revendications

1. Appareil de traitement (4) comprenant
un cathéter (1) qui inclut une tige d'administration (11), la tige d'administration (11) s'étendant dans une direction axiale (Z) et étant configurée pour contenir, dans une région d'extrémité de pointe (A1), une endoprothèse (2) ayant un diamètre réduit,
la tige d'administration (11) incluant
un élément en forme de tube (110) qui inclut une ou une pluralité de lumières (L) fournies dans la direction axiale (Z), et
une pluralité d'éléments centraux (112, 112a, 112b, 112c, 112d) qui s'étendent dans la direction axiale (Z) à l'intérieur de l'élément en forme de tube (110) au moins dans la région d'extrémité de pointe (A1), et qui ont une déformabilité plastique, la pluralité d'éléments centraux (112, 112a, 112b, 112c, 112d) étant disposés de manière sensiblement isotrope dans une direction circonférentielle externe (R1) de la lumière (L) à l'intérieur de l'élément en forme de tube (110) ; dans lequel
la tige d'administration (11) inclut en outre une couche de recouvrement (111) qui recouvre la lumière (L), et
la couche de recouvrement (111) et au moins l'un de la pluralité d'éléments centraux (112, 112a, 112b, 112c, 112d) sont en contact l'un avec l'autre.

2. Appareil de traitement (4) selon la revendication 1, dans lequel la pluralité d'éléments centraux (112, 112a, 112b, 112c, 112d) ont des diamètres respectifs (r2) qui sont environ égaux ou supérieurs à un diamètre (r(L)) de la lumière (L).

3. Appareil de traitement (4) selon l'une quelconque des revendications 1 ou 2, dans lequel la tige d'administration (11) inclut en outre une couche de renforcement (113), la couche de renforcement (113) étant fournie sur une surface circonférentielle externe de l'élément en forme de tube (110) dans une région (A2, A3) du côté d'extrémité de base de la région d'extrémité de pointe (A1), et ayant une ou une pluralité de fentes (S), et
la fente (S) est fournie dans une direction circonférentielle (R2) de la couche de renforcement (113) ou est fournie en spirale sur la couche de renforcement (113).

4. Appareil de traitement (4) selon l'une quelconque des revendications 1 à 3, dans lequel le nombre de la lumière (L) fournie est de un, et le nombre des éléments centraux (112, 112a, 112b, 112c, 112d) fournis est de quatre.

5. Appareil de traitement (4) selon l'une quelconque des revendications 1 à 4, comprenant en outre :
l'endoprothèse (2) ; et
un capot (3) qui contient, dans la région d'extrémité de pointe (A1), l'endoprothèse (2) ayant le diamètre réduit.
